(19) Europäisches Patentamt / European Patent Office / Office européen des brevets

(11) EP 3 842 808 A1

(12) EUROPEAN PATENT APPLICATION
published in accordance with Art. 153(4) EPC

(43) Date of publication:
30.06.2021 Bulletin 2021/26

(51) Int Cl.:
*G01N 33/72* (2006.01)

(21) Application number: 19852092.6

(22) Date of filing: 23.08.2019

(86) International application number:
PCT/JP2019/033057

(87) International publication number:
WO 2020/040294 (27.02.2020 Gazette 2020/09)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
KH MA MD TN

(30) Priority: 24.08.2018 JP 2018157561

(71) Applicant: NATIONAL UNIVERSITY
CORPORATION
YOKOHAMA NATIONAL UNIVERSITY
Yokohama-shi
Kanagawa 240-8501 (JP)

(72) Inventor: FUJIKAWA Tetsuya
Yokohama-shi, Kanagawa 240-8501 (JP)

(74) Representative: Arnold & Siedsma
Bezuidenhoutseweg 57
2594 AC The Hague (NL)

(54) ADMINISTRATION MANAGING APPARATUS, ADMINISTRATION MANAGING METHOD AND PROGRAM

(57) A target hemoglobin level of a patient; a current hemoglobin level of the patient; a specific decrease amount per unit period indicating an amount of decrease of a hemoglobin level of the patient when an erythropoiesis stimulating agent is not administered during the unit period; and a specific increase amount per unit period being an amount of increase of the hemoglobin level of the patient when an erythropoiesis stimulating agent at a maximum allowable unit amount per unit period is administered during the unit period and indicating a relative amount of increase in which the hemoglobin level when the erythropoiesis stimulating agent is not administered during the unit period is used as a reference are acquired. Furthermore, an amount of erythropoiesis stimulating agent to be administered to the patient per unit period is calculated on the basis of these pieces of information.

FIG. 1

EP 3 842 808 A1

**Description**

TECHNICAL FIELD

The present invention relates to an administration managing apparatus, an administration managing method, and a program for an erythropoiesis stimulating agent.

Priority is claimed on Japanese Patent Application No. 2018-157561, filed August 24, 2018, the content of which is incorporated herein by reference.

BACKGROUND ART

Many hemodialysis patients have renal anemia as a complication and renal anemia is one of the major prognostic factors. In actual clinical practice, when hemoglobin levels of patients are outside of target ranges, the attending physicians determine amounts of erythropoiesis stimulating agents (ESAs), which are therapeutic agents for renal anemia, to be administered and thus control the hemoglobin levels of the patients. Patent Document 1 discloses a technique for determining a hemoglobin production rate which is a target hemoglobin level of a patient from a red blood cell lifespan and determining a required amount of ESA to be administered.

[Prior Art Documents]

[Patent Documents]

[Patent Document 1]
Japanese Patent No. 6190632

SUMMARY OF THE INVENTION

Problem to be Solved by the Invention

Here, the technique of Patent Document 1 described above determines a hemoglobin production rate as something in which an amount of decrease per unit period of a hemoglobin level (hemoglobin concentration) of a patient per day is constant and determines a required amount of ESA to be administered. However, an amount of decrease in hemoglobin level per unit period differs for each patient and each of the physical characteristics of the patient. Furthermore, an amount of increase in hemoglobin level per unit period according to an amount of erythropoiesis stimulating agent to be administered differs for each patient and each of the physical characteristics of the patient and is affected by an amount of decrease in hemoglobin level per unit period. Therefore, there is a need for a technique in which a more appropriate amount of erythropoiesis stimulating agent to be administered per unit period according to the patient can be easily ascertained. Furthermore, as such a technique, a technique based on a technique for predicting an amount of change in the hemoglobin level per unit period in accordance with an amount of erythropoiesis stimulating agent to be administered per unit period on the basis of a new hemoglobin production reaction confirmed through reticulocyte hemoglobin measurement is required. However, there is no technique for predicting an amount of change in hemoglobin level.

Thus, an object of the present invention is to develop a technique for predicting an amount of change in hemoglobin level and to provide an administration managing apparatus, an administration managing method, and a program in which the above-described problems are solved.

Means for Solving the Problem

According to a first aspect of the present invention, an administration managing apparatus includes: an administration amount calculation unit calculates an amount of an erythropoiesis stimulating agent to be administered to a patient per unit period based on a target hemoglobin level of the patient, a current hemoglobin level of the patient, a specific decrease amount per unit period, and a specific increase amount per unit period, the specific decrease amount per unit period indicating an amount of decrease of a hemoglobin level of the patient when the erythropoiesis stimulating agent is not administered during the unit period, the specific increase amount per unit period being an amount of increase of the hemoglobin level of the patient when the erythropoiesis stimulating agent at a maximum allowable unit amount per unit period is administered during the unit period and indicating a relative amount of increase with a hemoglobin level when the erythropoiesis stimulating agent is not administered during the unit period serving as a reference.

In the above-mentioned administration managing apparatus, the administration amount calculation unit may calculate the amount of the erythropoiesis stimulating agent to be administered per unit period by multiplying a value obtained by adding the specific decrease amount per unit period to a difference between the target hemoglobin level and the current hemoglobin level of the patient by a unit amount of the erythropoiesis stimulating agent per amount of increase in hemoglobin level of the patient obtained by dividing the maximum allowable unit amount per unit period by the specific increase amount per unit period.

The above-mentioned administration managing apparatus may further include: an administration amount statistical value acquisition unit that acquires a statistical value of an amount of the erythropoiesis stimulating agent to be administered applied to the patient in a past during the unit period; a hemoglobin level change amount calculation unit that calculates an amount of change in hemoglobin level per unit period of the patient based on the statistical value, a specific decrease amount per unit period in the past of the patient, and a specific increase amount per unit period in the past of the patient; and a specific increase amount calculation unit that calculates a new specific increase amount per unit period based on the amount of change in hemoglobin level per unit period of the patient.

In the above-mentioned administration managing apparatus, the specific increase amount calculation unit may calculate a specific increase amount indicating a weighted average value in which the specific increase amount per unit period in the past is weighted more than the new specific increase amount per unit period using the specific increase amount per unit period in the past of the patient and the new specific increase amount per unit period.

In the above-mentioned administration managing apparatus, the hemoglobin level change amount calculation unit may calculate the amount of change in hemoglobin level per unit period of the patient by subtracting the specific decrease amount per unit period from a value obtained by multiplying a ratio of the statistical value of the amount of administration of the erythropoiesis stimulating agent per unit period to the maximum allowable unit amount per unit period of the erythropoiesis stimulating agent by the specific increase amount per unit period.

The above-mentioned administration managing apparatus may further include: a specific increase amount acquisition unit that acquires a specific increase amount identified based on a measurement result of a reticulocyte hemoglobin measurement as the specific increase amount per unit period, and the administration amount calculation unit may calculate the amount of the erythropoiesis stimulating agent to be administered to the patient per unit period using the acquired specific increase amount.

The above-mentioned administration managing apparatus may further include: a specific increase amount calculation unit that calculates the specific increase amount per unit period of the patient based on a specific increase amount calculation expression according to a physical characteristic group to which the patient belongs.

The above-mentioned administration managing apparatus may further include: a specific decrease amount calculation unit that calculates the specific decrease amount per unit period of the patient based on a specific decrease amount calculation expression according to a physical characteristic group to which the patient belongs.

The above-mentioned administration managing apparatus may further include: a specific decrease amount calculation unit that acquires clinical data when the erythropoiesis stimulating agent is not administered in a case in which the patient is in a good condition and calculates the specific decrease amount per unit period based on the clinical data.

According to a second aspect of the present invention, an administration managing method includes: calculating, by an administration managing apparatus, an amount of an erythropoiesis stimulating agent to be administered to a patient per unit period based on a target hemoglobin level of the patient, a current hemoglobin level of the patient, a specific decrease amount per unit period, and a specific increase amount per unit period, the specific decrease amount per unit period indicating an amount of decrease of a hemoglobin level of the patient when the erythropoiesis stimulating agent is not administered during the unit period, the specific increase amount per unit period being an amount of increase of the hemoglobin level of the patient when the erythropoiesis stimulating agent at a maximum allowable unit amount per unit period is administered during the unit period and indicating a relative amount of increase with a hemoglobin level when the erythropoiesis stimulating agent is not administered during the unit period serving as a reference.

According to a third aspect of the present invention, a program is a program that causes a computer of an administration managing apparatus to execute: calculating an amount of an erythropoiesis stimulating agent to be administered to a patient per unit period based on a target hemoglobin level of the patient, a current hemoglobin level of the patient, a specific decrease amount per unit period, and a specific increase amount per unit period, the specific decrease amount per unit period indicating an amount of decrease of a hemoglobin level of the patient when the erythropoiesis stimulating agent is not administered during the unit period, the specific increase amount per unit period being an amount of increase of the hemoglobin level of the patient when the erythropoiesis stimulating agent at a maximum allowable unit amount per unit period is administered during the unit period and indicating a relative amount of increase with a hemoglobin level when the erythropoiesis stimulating agent is not administered during the unit period serving as a reference.

Effect of the Invention

According to the present invention, it is possible to provide an administration managing apparatus in which a more appropriate amount of erythropoiesis stimulating agent to be administered per unit period according to a patient is calculated.

BRIEF DESCRIPTION OF THE DRAWINGS

Fig. 1 is a schematic diagram of an administration managing system including an administration managing apparatus according to an embodiment of the present invention.
Fig. 2 is a diagram illustrating a constitution of hardware of the administration managing apparatus according to the embodiment of the present invention.
Fig. 3 is a functional block diagram of the administration managing apparatus according to the embodiment of the present invention.
Fig. 4 is a first diagram illustrating a processing flow of the administration managing apparatus according to the embodiment of the present invention.
Fig. 5 is a second diagram illustrating a processing flow of the administration managing apparatus according to the embodiment of the present invention.
Fig. 6 is a functional block diagram of a modified example of the administration managing apparatus.
Fig. 7 is a diagram for explaining a specific decrease amount and a specific increase amount according to the embodiment of the present invention.

EMBODIMENTS FOR CARRYING OUT THE INVENTION

An administration managing apparatus according to an embodiment of the present invention will be described below with reference to the drawings.

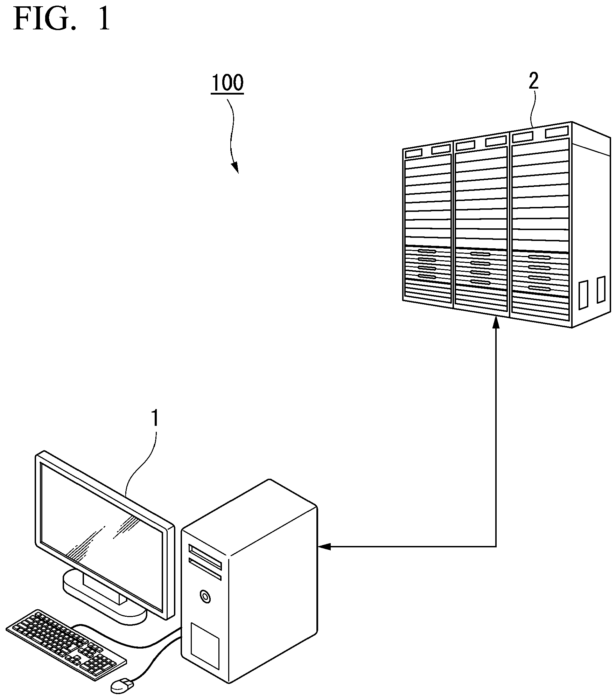

Fig. 1 is a schematic diagram of an administration managing system 100 including the administration managing apparatus 1 according to the embodiment.
As illustrated in Fig. 1, the administration managing apparatus 1 is a computer. The administration managing apparatus 1 may be able to be communicatively connected to an electronic medical record server 2 and to acquire electronic medical record information regarding a patient. The administration managing system 100 is constituted by communicatively connecting the administration managing apparatus 1 and the electronic medical record server 2.
The administration managing apparatus 1 according to the embodiment acquires a target hemoglobin level of a patient, a current hemoglobin level of the patient, and a specific decrease amount per unit period indicating an amount of decrease in hemoglobin level of the patient when an erythropoiesis stimulating agent is not administered during a unit period. Furthermore, the administration managing apparatus 1 acquires a specific increase amount per unit period which is a sum of an amount of increase in hemoglobin level of a patient with a current hemoglobin level of the patient serving as a reference when an erythropoiesis stimulating agent of a maximum allowable unit amount per unit period is administered during the unit period and a specific decrease amount per unit period. Moreover, the administration managing apparatus 1 calculates an amount of erythropoiesis stimulating agent to be administered to the patient per unit period using the target hemoglobin level of the patient, the current hemoglobin level of the patient, the specific decrease amount per unit period of the patient, and the specific increase amount per unit period of the patient. The "specific" in the terms of the specific decrease amount and the specific increase amount means that an amount of decrease and an amount of increase are specific for each patient (or a group to which the patient belongs).

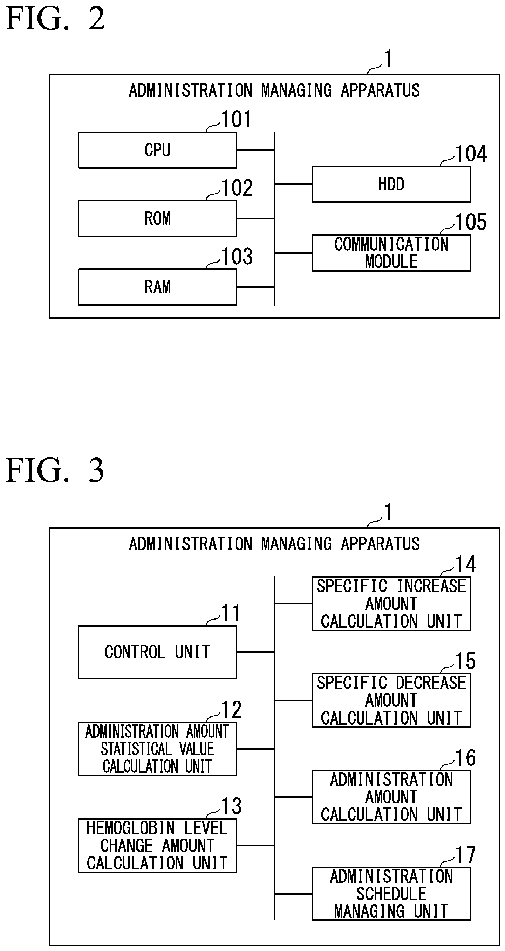

Fig. 2 is a diagram illustrating a constitution of hardware of the administration managing apparatus 1 according to the embodiment.
As illustrated in Fig. 2, the administration managing apparatus 1 is a computer including hardware such as a central processing unit (CPU) 101, a read only memory (ROM) 102, a random access memory (RAM) 103, a hard disk drive (HDD) 104, and a communication module 105.
Fig. 3 is a functional block diagram of the administration managing apparatus 1.
The administration managing apparatus 1 starts up when receiving an input of electric power and executes an administration managing program which has been stored in advance. Thus, the administration managing apparatus 1 has functions of a control unit 11, an administration amount statistical value calculation unit 12, a hemoglobin level change amount calculation unit 13, a specific increase amount calculation unit 14, a specific decrease amount calculation unit 15, an administration amount calculation unit 16, and an administration schedule managing unit 17.
The control unit 11 controls each functional unit of the administration managing apparatus 1.

The administration amount statistical value calculation unit 12 calculates a statistical value of an amount of erythropoiesis stimulating agent to be administered to a patient during a unit period in the past which has been performed during a unit period so that a hemoglobin level of the patient is made to reach the target hemoglobin level.

The hemoglobin level change amount calculation unit 13 calculates a predicted value of an amount of change in hemoglobin level per unit period of a patient on the basis of a statistical value of an amount of erythropoiesis stimulating agent to be administered to the patient per unit period in the past, a specific decrease amount per unit period in the past, and a specific increase amount per unit period in the past.

The specific increase amount calculation unit 14 calculates a specific increase amount per unit period for each patient.

The specific decrease amount calculation unit 15 calculates a specific decrease amount per unit period for each patient.

The administration amount calculation unit 16 calculates an amount of erythropoiesis stimulating agent to be administered to a patient per unit period using the target hemoglobin level of the patient, the current hemoglobin level of the patient, the specific decrease amount per unit period of the patient, and a specific increase amount per unit period of the patient.

The administration schedule managing unit 17 generates administration schedule information regarding an erythropoiesis stimulating agent to be administered to the patient on the basis of an amount of erythropoiesis stimulating agent to be administered to the patient per unit period.

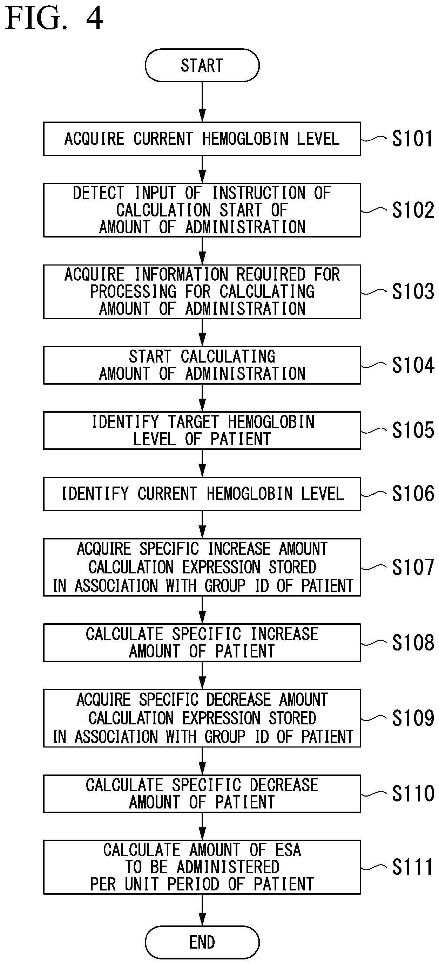

Fig. 4 is a first diagram illustrating a processing flow of the administration managing apparatus 1.

The processing flow of the administration managing apparatus 1 will be described below step by step.

For example, a doctor collects blood from a dialysis patient when subjecting the dialysis patient to dialysis. The doctor sends the blood of the dialysis patient to an inspector. Thus, the inspector measures a current hemoglobin level of the patient from a blood component of the patient. The inspector sends the hemoglobin level to the doctor. The doctor inputs the current hemoglobin level of the patient to the administration managing apparatus 1. The administration managing apparatus 1 collects the current hemoglobin level of the patient (Step S101).

The control unit 11 of the administration managing apparatus 1 transmits information such as identification information (ID) of a patient, a current hemoglobin level, and a date and time to the electronic medical record server 2. The electronic medical record server 2 stores information regarding a current hemoglobin level, a hemoglobin level for each blood sampling date in the past, each amount of erythropoiesis stimulating agent (hereinafter referred to as an "ESA") to be administered to a patient per unit period once or multiple times in the past, each specific decrease amount of the patient per unit period calculated once or multiple times in the past, each specific increase amount of the patient per unit period calculated once or multiple times in the past, an age, a gender, a body weight, and identification information of a group to which the patient belongs on the basis of physical characteristics (a group ID) in association with an ID of a patient. The group ID may be, for example, group identification information set on the basis of a degree of renal anemia, an age, a gender, a body weight, and the like among the physical characteristics of the user.

In the embodiment, an example of the ESA is erythropoietin (EPO). In this case, a unit period is one week. The unit period may vary depending on a type of ESA. For example, in the ESA which lasts for a long period of time and has an effect of stimulating erythrocyte hematopoiesis, a long unit period of 2 to 4 weeks is set. A specific decrease amount per unit period indicates an amount of decrease in hemoglobin level of a patient when an ESA is not administered during a unit period. A specific increase amount per unit period indicates an amount of increase under the assumption that there is no specific decrease amount in a hemoglobin level of the patient when an ESA of a maximum allowable unit amount per unit period is administered during a unit period (specific decrease amount+amount of increase from a measurement start time point of a hemoglobin level). That is to say, the specific increase amount per unit period is an amount of increase in hemoglobin level of the patient when the ESA of the maximum allowable unit amount per unit period is administered during a unit period and indicates a relative amount of increase with the hemoglobin level serving as a reference when an erythropoiesis stimulating agent is not administered during a unit period. These specific decrease amount and specific increase amount vary from patient to patient. For example, the specific decrease amount and the specific increase amount are indexes which differ depending on an age, a gender, a body weight, a blood volume, a red blood cell lifespan, a hematopoietic capacity, a residual blood in dialysis circuit, an amount of blood to be collected/blood collection frequency, and the like for each individual patient. When an ESA is an EPO, a maximum allowable unit amount which can be administered to a renal anemia patient undergoing dialysis per unit period is 9000 IU (IU=international unit) in Japan. That is to say, the doctor is allowed to administer an EPO of 0 to 9000 IU during a unit period, that is, 1 week, to the patient in Japan.

If receiving an input of a current hemoglobin level of a patient and receiving an instruction to start calculating an amount of administration from the doctor, the administration managing apparatus 1 detects an input of the instruction (Step S102). The control unit 11 of the administration managing apparatus 1 acquires information required for processing for calculating an amount of administration from the electronic medical record server 2 (Step S103). For example, the administration managing apparatus 1 acquires a hemoglobin level for each blood sampling date in the past, each amount of ESA to be administered per unit period which has been administered to a patient once or multiple times in the past, each specific decrease amount of the patient per unit period calculated once or multiple times in the past, each specific increase amount of the patient per unit period calculated once or multiple times in the past, an age, a gender, a body weight, and a group ID from the electronic medical record server 2 on the basis of the patient ID.

Also, the administration managing apparatus 1 starts calculating a new amount of ESA to be administered to the patient per unit period (Step S104). To be specific, first, a target hemoglobin level of the patient is identified (Step S105). For example, the doctor inputs the target hemoglobin level to the administration managing apparatus 1 and thus the control unit 11 acquires and identifies the target hemoglobin level. The administration managing apparatus 1 may store the target hemoglobin level in advance. As an example, the target hemoglobin level is 10.5 g/dl. Furthermore, the control unit 11 identifies the current hemoglobin level input by the doctor (Step S106). In addition, the control unit 11 instructs the specific increase amount calculation unit 14 to calculate a specific increase amount per unit period of the patient. The control unit 11 instructs the specific decrease amount calculation unit 15 to calculate a specific decrease amount per unit period of the patient. A case in which a specific decrease amount and a specific increase amount are obtained from an amount of change in hemoglobin level will be described below. When the specific decrease amount and the specific increase amount are obtained from the amount of change in hemoglobin level, it is possible to obtain the specific decrease amount and the specific increase amount with high accuracy.

The specific increase amount calculation unit 14 acquires a specific increase amount calculation expression to be stored in association with the group ID of the patient (Step S107). The specific increase amount calculation unit 14 calculates a specific increase amount of the patient using the specific increase amount calculation expression (Step S108). The specific increase amount calculation expression is a calculation expression identified using statistical processing from information of each patient in a physical characteristic group of a patient indicated by a group ID (a specific increase amount, an average amount of EPO to be administered per unit period, and the like). As an example, the specific increase amount calculation expression is shown using the following Expression (1). This calculation expression changes in accordance with the group.

[Expression 1]

$$\text{Specific increase amount per unit period (one week) (mg/dL)}$$
$$= (-0.0577 \times \text{average amount EPO to be administered per unit period}) + 1153.784$$
$$\cdots(1)$$

The specific decrease amount calculation unit 15 acquires a specific decrease amount calculation expression to be stored in association with a group ID of a patient (Step S109). The specific decrease amount calculation unit 15 calculates a specific decrease amount of the patient using the specific decrease amount calculation expression (Step S110). The specific decrease amount calculation expression is a calculation expression identified using statistical processing for information of each patient in a physical characteristic group of the patient indicated by the group ID (a specific decrease amount, an average amount of EPO to be administered per unit period, and the like). As an example, the specific decrease amount calculation expression is expressed by the following Expression (2). This calculation expression changes in accordance with the group.

[Expression 2]

$$\text{Specific decrease amount per unit period (one week) (mg/dL)}$$
$$= (0.0746 \times \text{average amount EPO to be administered per unit period}) + 61.184$$
$$\cdots(2)$$

Also, the administration amount calculation unit 16 multiplies a value obtained by adding the specific decrease amount per unit period of the patient to a difference between the target hemoglobin level and the current hemoglobin level of the patient by an ESA unit amount per amount of increase in hemoglobin level of the patient obtained by dividing the maximum allowable unit amount of the patient by the specific increase amount of the patient (refer to Expression (3)). Thus, the administration amount calculation unit 16 calculates an amount of ESA (EPO) to be administered per unit period of the patient (Step Sill). The ESA unit amount per amount of increase in hemoglobin level of the patient may be a value obtained by approximating a plurality of relationships between the specific increase amount of the patient and the amount of ESA to be administered in the past with a linear line.

[Expression 3]

$$\text{Amount of EPO to be administered per unit period (one week)}$$

$$=([\text{target hemoglobin level} - \text{current hemoglobin level}] + \text{specific decrease}$$

$$\text{amount}) \times \text{maximum allowable unit amount per unit period (90000 IU)} \div \text{specific increase amount}$$

$$\cdots (3)$$

Through the above processing, it is possible to calculate an amount of ESA to be administered to the patient per unit period. The amount of administration is an amount of administration per unit period calculated on the basis of a specific decrease amount and a specific increase amount per unit period of a hemoglobin level identified for the group identified on the basis of the physical characteristics of the patient. Therefore, the administration managing apparatus 1 can calculate a more appropriate amount of ESA to be administered per unit period according to the patient. Furthermore, when the amount of administration is displayed on a monitor of the administration managing apparatus 1, a doctor can easily grasp the more appropriate amount of ESA to be administered per unit period according to the patient.

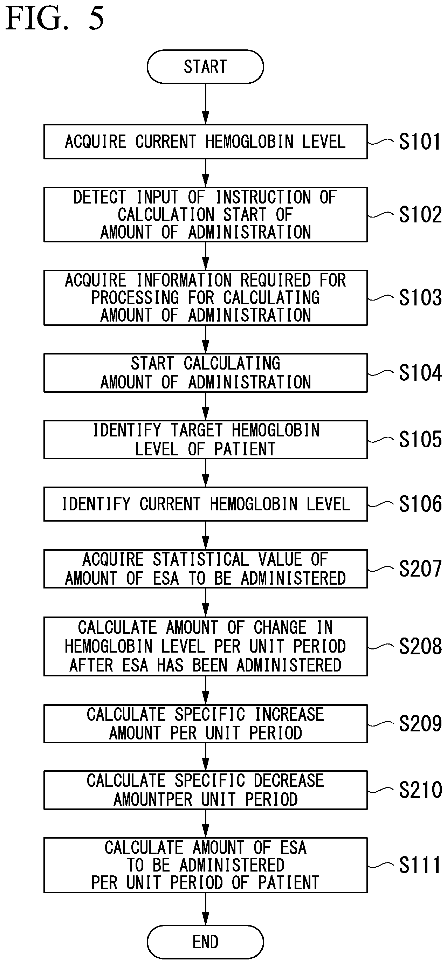

Fig. 5 is a second diagram illustrating the processing flow of the administration managing apparatus 1.

Although the specific increase amount and the specific decrease amount are calculated using the calculation expression according to the group to which the patient belongs in accordance with the physical characteristics in the above processing, the specific increase amount and the specific decrease amount may be identified or calculated through another method. The processing flow in this case will be described with reference to Fig. 5.

The processing from Step S101 to Step S106 is the same as that of the processing flow illustrated in Fig. 4. Furthermore, subsequently, the administration amount statistical value calculation unit 12 (an administration amount statistical value acquisition unit) acquires a statistical value of an amount of ESA to be administered to the patient applied in the past during a unit period to set the hemoglobin level of the patient to the target hemoglobin level (Step S207). The administration amount statistical value calculation unit 12 may calculate the statistical value. Alternatively, the administration amount statistical value calculation unit 12 may acquire the statistical value calculated in advance using the electronic medical record server 2 from the electronic medical record server 2. The calculation of the statistical value of the amount of administration may be, for example, an average value of one or more amounts to be administered per unit period in the past.

Also, the hemoglobin level change amount calculation unit 13 acquires a statistical value of an amount of ESA (EPO) to be administered to a patient per unit period in the past, a specific decrease amount per unit period of the patient in the past, and a specific increase amount per unit period of the patient in the past. These pieces of information are included in the information acquired from the electronic medical record server 2.

The hemoglobin level change amount calculation unit 13 may calculate an amount of change in hemoglobin level per unit period of a patient by multiplying a ratio of a statistical value of an amount of ESA to be administered per unit period to a maximum allowable unit amount of ESA per unit period by a specific increase amount per unit period and subtracting a specific decrease amount per unit period from that value. That is to say, the hemoglobin level change amount calculation unit 13 can calculate an amount of change in hemoglobin level per unit period after an ESA has been administered so as to reach the target hemoglobin level through Expression (4) using the statistical value of the amount of administration, the specific decrease amount, and the specific increase amount (Step S208).

[Expression 4]

$$\text{Hemoglobin level change amount after administration} =$$

$$\{\text{specific increase amount per unit period} \times (\text{statistical value of amount of}$$

$$\text{administration} \div \text{maximum allowable unit amount per unit period})\} - \text{specific decrease amount per}$$

$$\text{unit period} \qquad \cdots (4)$$

To be more specific, Expression (4) can be expressed by Expression (5) when an ESA is an EPO.

[Expression 5]

$$\text{Hemoglobin level change amount after administration} =$$

$$\{\text{specific increase amount per one week} \times (\text{statistical value of amount of EPO to be}$$

$$\text{administered} \div 9000 \text{ IU})\} - \text{specific decrease amount per one week} \qquad \cdots (5)$$

Also, the specific increase amount calculation unit 14 calculates a new specific increase amount per unit period on the basis of an actually measured amount of change in hemoglobin level per unit period of a patient (an amount of change in hemoglobin level after administration) and an amount of change in hemoglobin level calculated through the foregoing Expression (5) (an amount of change in hemoglobin level after administration) (Step S209). The specific increase amount calculation unit 14 calculates a specific increase amount on the basis of the actually measured amount of change in hemoglobin level (the amount of change in hemoglobin level after administration) in many cases. To be specific, since the amount of ESA (EPO) to be administered and the amount of change in hemoglobin level per unit period when the amount of administration is administrated can be grasped, it is possible to calculate a new specific increase amount per unit period through Expression (6). The specific increase amount is a specific increase amount specific to a patient.
[Expression 6]

$$\text{New specific increase amount per unit period}$$
$$=(\text{hemoglobin level change amount after administration}+\text{specific decrease amount})$$
$$\times(9000 \text{ IU}\div\text{actual amount of ESA to be administered per unit period}) \quad \cdots(6)$$

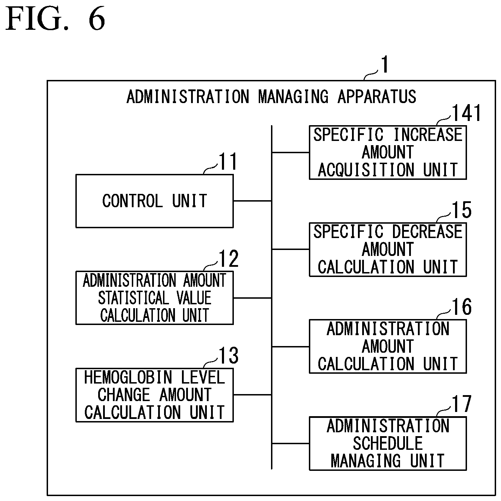

As illustrated in Fig. 6, the administration managing apparatus 1 may include a specific increase amount acquisition unit 141 instead of the specific increase amount calculation unit 14 and the specific increase amount acquisition unit 141 may acquire a value of a specific increase amount recorded in association with a patient ID from the electronic medical record server 2. For example, as the specific increase amount, a value identified on the basis of the measurement result of the reticulocyte hemoglobin measurement may be recorded in the electronic medical record server 2.

In the reticulocyte hemoglobin measurement, a new amount of hemoglobin to be produced can be measured, an amount of hemoglobin to be produced when there is no hematopoietic stimulation is defined as an amount of basal hemoglobin to be produced, and an amount of relative increase from an amount of basal hemoglobin to be produced when there is a hematopoietic stimulation is defined as an amount of reactive hemoglobin to be produced. The amount of reactive hemoglobin to be produced highly correlates with an ESA concentration. A specific decrease amount obtained through a normal hemoglobin level inspection inversely correlates with the amount of basal hemoglobin to be produced. An amount of relative increase in hemoglobin through ESA administration with respect to a specific decrease amount in the normal hemoglobin level inspection corresponds to an amount of reactive hemoglobin to be produced and highly correlates with the amount of ESA to be administered. The amount of relative increase in hemoglobin coincides with the specific increase amount when the maximum allowable unit amount of an erythropoiesis stimulating agent is administered during a unit period. An error is produced in a single measurement of the specific increase amount and the specific decrease amount. For this reason, it is possible to reduce an influence of an error by repeatedly measuring and averaging the specific increase amount and the specific decrease amount.

The specific decrease amount calculation unit 15 calculates a new specific decrease amount per unit period using the foregoing Expression (2) (Step S210).

The specific decrease amount calculation unit 15 may receive an input of a value observed in the actual clinical practice of the specific decrease amount and utilize the received specific decrease amount. For example, since it is assumed that an amount of increase in hemoglobin level per unit period is relatively large when an ESA is administered in a case in which a patient is in a good condition, a doctor may determine to suspend the administration of the ESA to the patient. At this time, the ESA may not be administered, an amount of decrease in hemoglobin level per unit period may be observed, and the value may be estimated as a specific decrease amount. A specific decrease amount based on the observation results of the actual clinical practice is a specific decrease amount specific to a patient. As another method, the specific decrease amount calculation unit 15 may acquire clinical data including daily hemoglobin levels of a patient in a good condition and with discontinuation of administration of an ESA and calculate a specific decrease amount per unit period on the basis of the clinical data.

The specific increase amount calculation unit 14 and the specific decrease amount calculation unit 15 may calculate a weighted average in which the specific increase amount and the specific decrease amount per unit period in the past are weighted more than the new specific increase amount and specific decrease amount per unit period using the specific increase amount and the specific decrease amount per unit period of the patient in the past and the new specific increase amount and specific decrease amount per unit period and utilize these values as the new specific increase amount and specific decrease amount per unit period of the patient. In this case, for example, the specific increase amount calculation unit 14 calculates a new specific increase amount per unit period through Expression (7).
[Expression 7]

New specific increase amount per unit period

=specific increase amount per unit period in past×(14/15)

+new specific increase amount calculated through Expression (6)×(1/15)

$$\cdots(7)$$

Alternatively, for example, the specific decrease amount calculation unit 15 calculates a new specific decrease amount per unit period through Expression (8).
[Expression 8]

New specific decrease amount per unit period

=specific decrease amount per unit period in past×(14/15)

+new specific decrease amount×(1/15)    $$\cdots(8)$$

The specific increase amount calculation unit 14 may calculate a new specific increase amount per unit period through Expression (9) using a method of a modified moving average (MMA).
[Expression 9]

New specific increase amount per unit period

=[specific increase amount per unit period in past×(MMA coefficient−1)+new specific

increase amount]÷MMA coefficient    $$\cdots(9)$$

The specific decrease amount calculation unit 15 may calculate a new specific decrease amount per unit period through Expression (10) using a method of a modified moving average (MMA).
[Expression 10]

New specific decrease amount per unit period

=[specific decrease amount per unit period in past×(MMA coefficient−1)+new specific

decrease amount]÷MMA coefficient    $$\cdots(10)$$

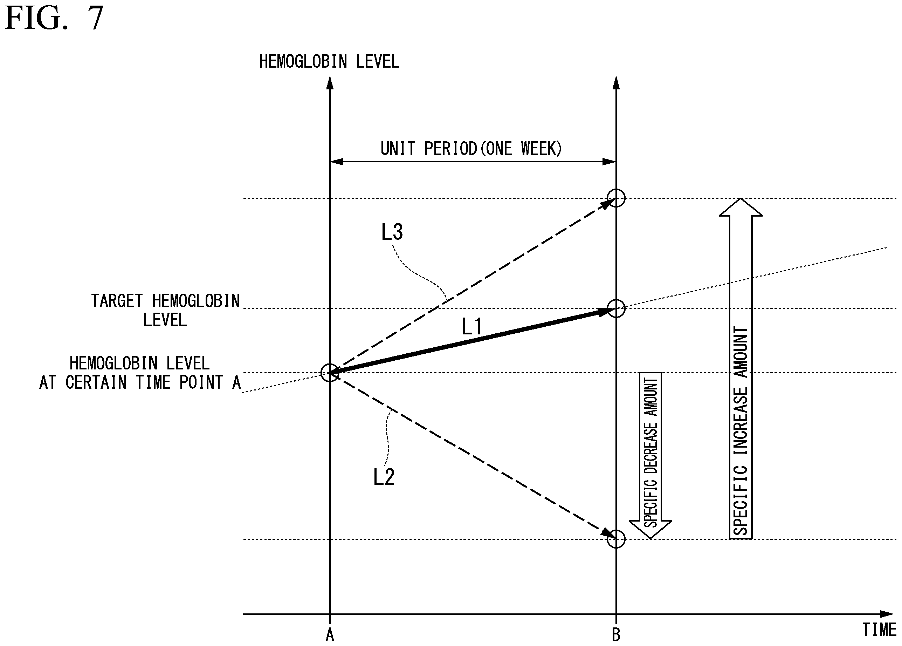

Fig. 7 is a diagram for explaining a specific decrease amount and a specific increase amount.
When a current hemoglobin level of a patient at a certain time point A falls below a target hemoglobin level, a doctor administers an ESA so that a hemoglobin level is a target hemoglobin level at a time point B after one week which is a unit period, from the time point A. It is desirable that this administration improve a hemoglobin level of a patient during a unit period as illustrated in an arrow L1 in Fig. 7 and the hemoglobin level reach a target hemoglobin level. The arrow L1 in Fig. 7 is an example. When a patient is in a bad physical condition or a large difference is produced between the hemoglobin level and the target hemoglobin level, it is desirable for the hemoglobin level of the patient to reach the vicinity of the target hemoglobin level at an early stage. Furthermore, although not illustrated in Fig. 7, when the hemoglobin level of the patient significantly exceeds the target hemoglobin level, measures such as discontinuing administration of an ESA are performed.
L2 illustrates an example of changes in hemoglobin level of a patient when an ESA is not administered during a unit period. An amount of hemoglobin level decreasing between the time point A which is a starting point of a unit period at the time of non-administration and the time point B after the unit period is defined as a specific decrease amount.
Also, L3 illustrates an example of changes in hemoglobin level of a patient when a maximum allowable unit amount of ESA per unit period is administered. When the maximum allowable unit amount of ESA per unit period is administered, an amount obtained by adding an amount of hemoglobin level increasing between the time point A which is a starting point of a unit period and the time point B after the unit period to a value of the specific decrease amount is defined as a specific increase amount.
The specific increase amount calculation unit 14 and the specific decrease amount calculation unit 15 may acquire a specific increase amount and a specific decrease amount through any of the above-described methods and calculate a specific increase amount and a specific decrease amount of each patient through machine learning by utilizing the specific increase amount and the specific decrease amount in the past and the new specific increase amount and specific decrease amount and reflecting a difference between individuals and a difference within individuals.

The administration amount calculation unit 16 multiplies a value obtained by adding a specific decrease amount per unit period of a patient to a difference between a target hemoglobin level and a current hemoglobin level of the patient by an ESA unit amount per amount of increase in hemoglobin level of the patient obtained by dividing a maximum allowable unit amount of the patient by a specific increase amount of the patient (refer to Expression (3)). Thus, the administration amount calculation unit 16 calculates a new amount of ESA (EPO) to be administered per unit period in the future associated with the patient (Step S111). Furthermore, the administration managing apparatus 1 repeatedly performs the above-described processing at the time of calculating the amount of ESA to be administered on the basis of data newly accumulated in the electronic medical record server 2 or the like.

When the calculated amount of administration exceeds the maximum allowable unit amount of 9000 IU per unit period, the administration amount calculation unit 16 identifies the maximum allowable unit amount of 9000 IU as an ESA (EPO) per unit period of the patient in the future.

When the calculated amount of administration is less than a minimum amount of administration set for an individual patient, the administration amount calculation unit 16 may identify the minimum amount of administration as an amount of ESA (EPO) to be administered per unit period of the patient in the future.

When the input current hemoglobin level is close to the target hemoglobin which falls within a prescribed range having the target hemoglobin level as a reference, the control unit 11 of the administration managing apparatus 1 may not calculate a new amount of ESA (EPO) to be administered per unit period in the future associated with the patient. In this case, the control unit 11 of the administration managing apparatus 1 may identify the calculated amount of administration for the patient last time as the amount of ESA (EPO) to be administered per unit period in the future associated with the patient.

Also, the administration schedule managing unit 17 generates administration schedule information indicating the calculated amount of ESA to be administered per unit period of the patient in the future and outputs the generated administration schedule information to the monitor of the administration managing apparatus 1. Thus, a doctor can confirm the amount of ESA to be administered per unit period of the patient in the future and administer the ESA based on the amount of ESA to be administered per unit period under the determination of the doctor.

According to the aforementioned processing, it is possible to calculate and output the appropriate amount of ESA to be administered per unit period based on a specific increase amount and a specific decrease amount of an individual patient. Furthermore, a doctor can confirm the administration schedule information and easily perform ESA administration based on the amount of ESA to be administered per unit period according to the patient.

Also, according to the aforementioned processing of the administration managing apparatus, it is possible to calculate and present the amount of ESA to be administered without the need for special additional inspections.

When the hemoglobin level is stabilized through ESA administration using an amount of administration in which there is no excessiveness and deficiency, it is possible to reduce a frequency of adjusting the amount of ESA to be administered, improve the prognosis of the patient, and reduce the medical work of a doctor, a nursing staff, or the like.

Furthermore, it is possible to reduce a calculation error of the amount of administration and a recording error in the electronic medical record or the like through the aforementioned processing.

In addition, it is possible to prevent an excessive administration by appropriately calculating an amount of administration according to a patient and medical costs can be expected to be reduced by reducing an amount of ESA to be administered.

The administration schedule managing unit 17 determines an amount of distribution on the basis of the new amount of ESA (EPO) to be administered per unit period in the future associated with the patient calculated through the aforementioned processing. For example, containers containing ESA drug solutions are managed to have different sizes and the volumes of the drug solutions differ. A doctor needs to determine the details of sizes of the containers and times at which the containers are utilized at the time of administrating the drug solutions on the basis of the amount of administration to the patient per unit period, the number of scheduled visits of visiting the hospital per unit period, and a difference between the current hemoglobin level of the patient and the target hemoglobin level. Usually, all of the drug solutions in the containers are administered when visiting the hospital once.

In such a case, for example, the administration schedule managing unit 17 determines whether the patient is in a good condition or in a bad condition on the basis of the difference between the current hemoglobin level of the patient and the target hemoglobin level. The administration schedule managing unit 17 may determine that the patient is in a bad condition if the difference between the current hemoglobin level of the patient and the target hemoglobin level is a prescribed threshold value or more. Furthermore, if the current hemoglobin level and a predicted hemoglobin level are a prescribed threshold value or more, similarly, it may be determined that the patient is in a bad condition. When determining that the patient is in a bad condition, the administration schedule managing unit 17 may emit an alert, distribute the amount of administration during a unit period to the number of scheduled visits to the hospital, and perform a distribution so that a large amount of ESA is to be administered at an early stage.

The administration schedule managing unit 17 determines a size of drug solution container used for each scheduled number of visits to the hospital per unit period using a distribution managing calculation expression for a prescribed amount of administration. The administration schedule managing unit 17 generates administration schedule information so that ESA administration is performed every one or two days when hemodialysis treatment is performed three times a week. However, in the generation of the administration schedule information, when an administration interval increases, the administration schedule managing unit 17 may determine a size of a drug solution container so that an amount of administration increases at a scheduled date of visiting the hospital before the administration interval increases such that a hemoglobin level does not decrease during the increased administration interval.

The administration schedule managing unit 17 may determine a size of a drug solution container so that the same amount of ESA can be administered for each scheduled date of visiting the hospital when it is determined that the patient is not in a bad condition on the basis of the current hemoglobin level of the patient. Furthermore, the administration schedule managing unit 17 may perform setting so that an amount of administration undergoes unbalanced allocation such that a large amount of ESA can be first administered to reach a target hemoglobin level at an early stage. Although various sizes of drug solution containers determined for each scheduled date of visiting the hospital are provided through a combination of an amount of administration per unit period, a capacity of a drug solution container, a state of a patient at the time of calculation, scheduled number of visits to the hospital per unit period, and the like in accordance with a type of ESA, the size of the drug solution container at each scheduled date of visiting the hospital is determined using a prescribed algorithm.

The aforementioned administration managing apparatus 1 includes a computer system therein. Furthermore, a program for causing the administration managing apparatus 1 to perform each of the aforementioned processes is stored in a computer-readable recording medium of the administration managing apparatus 1 and the aforementioned processing is performed by reading and executing this program using the computer of the administration managing apparatus 1. Here, the computer-readable recording medium refers to magnetic disks, magneto-optical disks, CD-ROMs, DVD-ROMs, semiconductor memories, and the like. Furthermore, this computer program may be distributed to a computer through a communication circuit and the computer receiving the distribution may execute the program.

Also, the aforementioned program may be for realizing a part of the functions of the aforementioned processing units. Furthermore, the program may be a so-called difference file (difference program) which can realize the aforementioned functions in combination with a program already recorded in the computer system.

INDUSTRIAL APPLICABILITY

The present invention may be applied to an administration managing apparatus, an administration managing method, and a program of an erythropoiesis stimulating agent.

Reference Symbol

1 Administration managing apparatus
2 Electronic medical record server
11 Control unit
12 Administration amount statistical value calculation unit
13 Hemoglobin level change amount calculation unit
14 Specific increase amount calculation unit
15 Specific decrease amount calculation unit
16 Administration amount calculation unit
17 Administration schedule managing unit

**Claims**

1. An administration managing apparatus, comprising:
   an administration amount calculation unit calculates an amount of an erythropoiesis stimulating agent to be administered to a patient per unit period based on a target hemoglobin level of the patient, a current hemoglobin level of the patient, a specific decrease amount per unit period, and a specific increase amount per unit period, the specific decrease amount per unit period indicating an amount of decrease of a hemoglobin level of the patient when the erythropoiesis stimulating agent is not administered during the unit period, the specific increase amount per unit period being an amount of increase of the hemoglobin level of the patient when the erythropoiesis stimulating agent at a maximum allowable unit amount per unit period is administered during the unit period and indicating a relative amount of increase with a hemoglobin level when the erythropoiesis stimulating agent is not administered during the unit period serving as a reference.

2. The administration managing apparatus according to claim 1, wherein the administration amount calculation unit calculates the amount of the erythropoiesis stimulating agent to be administered per unit period by multiplying a value obtained by adding the specific decrease amount per unit period to a difference between the target hemoglobin level and the current hemoglobin level of the patient by a unit amount of the erythropoiesis stimulating agent per amount of increase in hemoglobin level of the patient obtained by dividing the maximum allowable unit amount per unit period by the specific increase amount per unit period.

3. The administration managing apparatus according to claim 1 or 2, further comprising:

   an administration amount statistical value acquisition unit that acquires a statistical value of an amount of the erythropoiesis stimulating agent to be administered applied to the patient in a past during the unit period;
   a hemoglobin level change amount calculation unit that calculates an amount of change in hemoglobin level per unit period of the patient based on the statistical value, a specific decrease amount per unit period in the past of the patient, and a specific increase amount per unit period in the past of the patient; and
   a specific increase amount calculation unit that calculates a new specific increase amount per unit period based on the amount of change in hemoglobin level per unit period of the patient.

4. The administration managing apparatus according to claim 3, wherein the specific increase amount calculation unit calculates a specific increase amount indicating a weighted average value in which the specific increase amount per unit period in the past is weighted more than the new specific increase amount per unit period using the specific increase amount per unit period in the past of the patient and the new specific increase amount per unit period.

5. The administration managing apparatus according to claim 3 or 4, wherein the hemoglobin level change amount calculation unit calculates the amount of change in hemoglobin level per unit period of the patient by subtracting the specific decrease amount per unit period from a value obtained by multiplying a ratio of the statistical value of the amount of administration of the erythropoiesis stimulating agent per unit period to the maximum allowable unit amount per unit period of the erythropoiesis stimulating agent by the specific increase amount per unit period.

6. The administration managing apparatus according to claim 1 or 2, further comprising:

   a specific increase amount acquisition unit that acquires a specific increase amount identified based on a measurement result of a reticulocyte hemoglobin measurement as the specific increase amount per unit period, wherein the administration amount calculation unit calculates the amount of the erythropoiesis stimulating agent to be administered to the patient per unit period using the acquired specific increase amount.

7. The administration managing apparatus according to claim 1 or 2, further comprising:
   a specific increase amount calculation unit that calculates the specific increase amount per unit period of the patient based on a specific increase amount calculation expression according to a physical characteristic group to which the patient belongs.

8. The administration managing apparatus according to any one of claims 1 to 7, further comprising:
   a specific decrease amount calculation unit that calculates the specific decrease amount per unit period of the patient based on a specific decrease amount calculation expression according to a physical characteristic group to which the patient belongs.

9. The administration managing apparatus according to any one of claims 1 to 7, further comprising:
   a specific decrease amount calculation unit that acquires clinical data when the erythropoiesis stimulating agent is not administered in a case in which the patient is in a good condition and calculates the specific decrease amount per unit period based on the clinical data.

10. The administration managing apparatus according to any one of claims 1 to 7, further comprising:
    a specific decrease amount calculation unit that calculates a specific decrease amount indicating an average value in which a specific decrease amount per unit period in a past is weighted more than a new specific decrease amount per unit period using the specific decrease amount per unit period in the past of the patient and the new specific decrease amount per unit period.

11. An administration managing method, comprising:

calculating, by an administration managing apparatus, an amount of an erythropoiesis stimulating agent to be administered to a patient per unit period based on a target hemoglobin level of the patient, a current hemoglobin level of the patient, a specific decrease amount per unit period, and a specific increase amount per unit period, the specific decrease amount per unit period indicating an amount of decrease of a hemoglobin level of the patient when the erythropoiesis stimulating agent is not administered during the unit period, the specific increase amount per unit period being an amount of increase of the hemoglobin level of the patient when the erythropoiesis stimulating agent at a maximum allowable unit amount per unit period is administered during the unit period and indicating a relative amount of increase with a hemoglobin level when the erythropoiesis stimulating agent is not administered during the unit period serving as a reference.

12. A program causing a computer of an administration managing apparatus to execute:

calculating an amount of an erythropoiesis stimulating agent to be administered to a patient per unit period based on a target hemoglobin level of the patient, a current hemoglobin level of the patient, a specific decrease amount per unit period, and a specific increase amount per unit period, the specific decrease amount per unit period indicating an amount of decrease of a hemoglobin level of the patient when the erythropoiesis stimulating agent is not administered during the unit period, the specific increase amount per unit period being an amount of increase of the hemoglobin level of the patient when the erythropoiesis stimulating agent at a maximum allowable unit amount per unit period is administered during the unit period and indicating a relative amount of increase with a hemoglobin level when the erythropoiesis stimulating agent is not administered during the unit period serving as a reference.

FIG. 1

# FIG. 2

ADMINISTRATION MANAGING APPARATUS — 1

CPU — 101

ROM — 102

RAM — 103

HDD — 104

COMMUNICATION MODULE — 105

# FIG. 3

ADMINISTRATION MANAGING APPARATUS — 1

CONTROL UNIT — 11

ADMINISTRATION AMOUNT STATISTICAL VALUE CALCULATION UNIT — 12

HEMOGLOBIN LEVEL CHANGE AMOUNT CALCULATION UNIT — 13

SPECIFIC INCREASE AMOUNT CALCULATION UNIT — 14

SPECIFIC DECREASE AMOUNT CALCULATION UNIT — 15

ADMINISTRATION AMOUNT CALCULATION UNIT — 16

ADMINISTRATION SCHEDULE MANAGING UNIT — 17

# FIG. 4

```
        START
          │
          ▼
ACQUIRE CURRENT HEMOGLOBIN LEVEL          ─── S101
          │
          ▼
DETECT INPUT OF INSTRUCTION OF
CALCULATION START OF                      ─── S102
AMOUNT OF ADMINISTRATION
          │
          ▼
ACQUIRE INFORMATION REQUIRED FOR
PROCESSING FOR CALCULATING                ─── S103
AMOUNT OF ADMINISTRATION
          │
          ▼
START CALCULATING
AMOUNT OF ADMINISTRATION                  ─── S104
          │
          ▼
IDENTIFY TARGET HEMOGLOBIN
LEVEL OF PATIENT                          ─── S105
          │
          ▼
IDENTIFY CURRENT HEMOGLOBIN LEVEL         ─── S106
          │
          ▼
ACQUIRE SPECIFIC INCREASE AMOUNT
CALCULATION EXPRESSION STORED             ─── S107
IN ASSOCIATION WITH GROUP ID OF PATIENT
          │
          ▼
CALCULATE SPECIFIC INCREASE
AMOUNT OF PATIENT                         ─── S108
          │
          ▼
ACQUIRE SPECIFIC DECREASE AMOUNT
CALCULATION EXPRESSION STORED             ─── S109
IN ASSOCIATION WITH GROUP ID OF PATIENT
          │
          ▼
CALCULATE SPECIFIC DECREASE
AMOUNT OF PATIENT                         ─── S110
          │
          ▼
CALCULATE AMOUNT OF ESA
TO BE ADMINISTERED                        ─── S111
PER UNIT PERIOD OF PATIENT
          │
          ▼
        END
```

# FIG. 5

```
               ┌─────────────┐
               │    START    │
               └─────────────┘
                      │
                      ▼
    ┌──────────────────────────────────────┐
    │   ACQUIRE CURRENT HEMOGLOBIN LEVEL    │─── S101
    └──────────────────────────────────────┘
                      │
                      ▼
    ┌──────────────────────────────────────┐
    │    DETECT INPUT OF INSTRUCTION OF     │
    │          CALCULATION START OF         │─── S102
    │        AMOUNT OF ADMINISTRATION       │
    └──────────────────────────────────────┘
                      │
                      ▼
    ┌──────────────────────────────────────┐
    │     ACQUIRE INFORMATION REQUIRED FOR  │
    │       PROCESSING FOR CALCULATING      │─── S103
    │        AMOUNT OF ADMINISTRATION       │
    └──────────────────────────────────────┘
                      │
                      ▼
    ┌──────────────────────────────────────┐
    │            START CALCULATING          │
    │        AMOUNT OF ADMINISTRATION       │─── S104
    └──────────────────────────────────────┘
                      │
                      ▼
    ┌──────────────────────────────────────┐
    │        IDENTIFY TARGET HEMOGLOBIN     │
    │            LEVEL OF PATIENT           │─── S105
    └──────────────────────────────────────┘
                      │
                      ▼
    ┌──────────────────────────────────────┐
    │     IDENTIFY CURRENT HEMOGLOBIN LEVEL │─── S106
    └──────────────────────────────────────┘
                      │
                      ▼
    ┌──────────────────────────────────────┐
    │        ACQUIRE STATISTICAL VALUE OF   │
    │     AMOUNT OF ESA TO BE ADMINISTERED  │─── S207
    └──────────────────────────────────────┘
                      │
                      ▼
    ┌──────────────────────────────────────┐
    │      CALCULATE AMOUNT OF CHANGE IN    │
    │     HEMOGLOBIN LEVEL PER UNIT PERIOD  │─── S208
    │      AFTER ESA HAS BEEN ADMINISTERED  │
    └──────────────────────────────────────┘
                      │
                      ▼
    ┌──────────────────────────────────────┐
    │          CALCULATE SPECIFIC INCREASE  │
    │            AMOUNT PER UNIT PERIOD     │─── S209
    └──────────────────────────────────────┘
                      │
                      ▼
    ┌──────────────────────────────────────┐
    │          CALCULATE SPECIFIC DECREASE  │
    │           AMOUNTPER UNIT PERIOD       │─── S210
    └──────────────────────────────────────┘
                      │
                      ▼
    ┌──────────────────────────────────────┐
    │           CALCULATE AMOUNT OF ESA     │
    │            TO BE ADMINISTERED         │─── S111
    │        PER UNIT PERIOD OF PATIENT     │
    └──────────────────────────────────────┘
                      │
                      ▼
               ┌─────────────┐
               │     END     │
               └─────────────┘
```

# FIG. 6

ADMINISTRATION MANAGING APPARATUS — 1

SPECIFIC INCREASE AMOUNT ACQUISITION UNIT — 141

CONTROL UNIT — 11

SPECIFIC DECREASE AMOUNT CALCULATION UNIT — 15

ADMINISTRATION AMOUNT STATISTICAL VALUE CALCULATION UNIT — 12

ADMINISTRATION AMOUNT CALCULATION UNIT — 16

HEMOGLOBIN LEVEL CHANGE AMOUNT CALCULATION UNIT — 13

ADMINISTRATION SCHEDULE MANAGING UNIT — 17

FIG. 7

## INTERNATIONAL SEARCH REPORT

International application No.

PCT/JP2019/033057

A.    CLASSIFICATION OF SUBJECT MATTER
Int.Cl. G01N33/72(2006.01)i

According to International Patent Classification (IPC) or to both national classification and IPC

B.    FIELDS SEARCHED

Minimum documentation searched (classification system followed by classification symbols)
Int.Cl. G01N33/72

Documentation searched other than minimum documentation to the extent that such documents are included in the fields searched

| | |
|---|---|
| Published examined utility model applications of Japan | 1922–1996 |
| Published unexamined utility model applications of Japan | 1971–2019 |
| Registered utility model specifications of Japan | 1996–2019 |
| Published registered utility model applications of Japan | 1994–2019 |

Electronic data base consulted during the international search (name of data base and, where practicable, search terms used)
JSTPlus/JMEDPlus/JST7580 (JDreamIII)

C.    DOCUMENTS CONSIDERED TO BE RELEVANT

| Category* | Citation of document, with indication, where appropriate, of the relevant passages | Relevant to claim No. |
|---|---|---|
| A | US 2017/0128532 A1 (HOFFMANN-LA ROCHE, INC.) 11 May 2017 & WO 2015/193462 A2 & EP 2957293 A1 | 1–12 |
| A | US 2014/0200181 A1 (FUERTINGER, D. H.) 17 July 2014 & WO 2013/036836 A2 | 1–12 |
| A | WO 2014/200054 A1 (NIPRO CORP.) 18 December 2014 & US 2016/0085939 A1 & EP 3009843 A1 & CN 105283766 A | 1–12 |

☒   Further documents are listed in the continuation of Box C.          ☐   See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 31 October 2019 (31.10.2019) | 12 November 2019 (12.11.219) |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

## INTERNATIONAL SEARCH REPORT

| | International application No. |
|---|---|
| | PCT/JP2019/033057 |

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2013-516680 A (MAYO FOUNDATION FOR MEDICAL EDUCATION AND RESEARCH) 13 May 2013 & US 2013/0191097 A1 & WO 2011/082421 A1 | 1-12 |
| A | 新里高弘，柴田和彦，　目標ヘモグロビン濃度を達成するESA投与量の決定のための新しいアルゴリズム，腎と透析，January 2018, vol. 84, no. 1, pp. 124-130, non-official translation (SHINZATO, Takahiro, SHIBATA, Kazuhiko, "A new algorithm for determining ESA dosage to achieve target hemoglobin concentration", Kidney and dialysis) | 1-12 |
| P,A | WO 2018/229739 A1 (NIPRO CORP.) 20 December 2018 & JP 2019-2898 A | 1-12 |
| P,A | 藤川哲也 他，赤血球造血刺激因子製剤の新規投与量算出法の評価，日本透析医学会雑誌，28 May 2019, vol. 52, supplement 1, p. 463, non-official translation (FUJIKAWA, Tetsuya, "Evaluation of new dose calculation method for erythropoiesis stimulating agent", Nihon Toseki Igakkai Zasshi) | 1-12 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2018157561 A

- JP 6190632 B